(19) Europäisches Patentamt
European Patent Office
Office européen des brevets

(11) Veröffentlichungsnummer: **0 218 541**
**B1**

# (12) EUROPÄISCHE PATENTSCHRIFT

(45) Veröffentlichungstag der Patentschrift: **22.11.90**

(21) Anmeldenummer: **86730155.8**

(22) Anmeldetag: **03.10.86**

(51) Int. Cl.$^5$: **C 07 D 471/04,** A 61 K 31/435 // (C07D471/04, 221:00, 209:00)

## (54) 3-Vinyl- und 3-Ethinyl-beta-carbolinderivate, ihre Herstellung und ihre Verwendung als Arzneimittel.

(30) Priorität: **04.10.85 DE 3535927**

(43) Veröffentlichungstag der Anmeldung:
**15.04.87 Patentblatt 87/16**

(45) Bekanntmachung des Hinweises auf die Patenterteilung:
**22.11.90 Patentblatt 90/47**

(84) Benannte Vertragsstaaten:
**AT BE CH DE ES FR GB GR IT LI LU NL SE**

(56) Entgegenhaltungen:
**EP-A-0 054 507**
**EP-A-0 110 814**
**EP-A-0 137 390**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen**
**Müllerstrasse 170/178 Postfach 65 03 11**
**D-1000 Berlin 65 (DE)**

(72) Erfinder: **Seidelmann, Dieter, Dr.**
**Stierstrasse 14**
**D-1000 Berlin 41 (DE)**
Erfinder: **Huth, Andreas, Dr.**
**Kirchweg 55**
**D-1000 Berlin 38 (DE)**
Erfinder: **Schmiechen, Ralph, Dr.**
**Bayernring 27**
**D-1000 Berlin 42 (DE)**
Erfinder: **Wiechert, Rudolf, Prof. Dr.**
**Petzowerstrasse 8a**
**D-1000 Berlin 39 (DE)**
Erfinder: **Schneider, Herbert, Dr.**
**Duisburgerstrasse 20**
**D-1000 Berlin 15 (DE)**
Erfinder: **Stephens, David Norman, Dr.**
**Hildegardstrasse 16a**
**D-1000 Berlin 31 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 218 541 B1

**Beschreibung**

Die Erfindung betrifft neue 3-Vinyl- und 3-Ethinyl-β-carbolinderivate, ihre Herstellung und ihre Verwendung als Arzneimittel.

Die erfindungsgemäßen Verbindungen besitzen wertvolle pharmakologische Eigenschaften. Sie beeinflussen insbesondere das Zentralnervensystem und eignen sich somit als Psychopharmaka.

Die erfindungsgemäßen Verbindungen haben die allgemeine Formel I

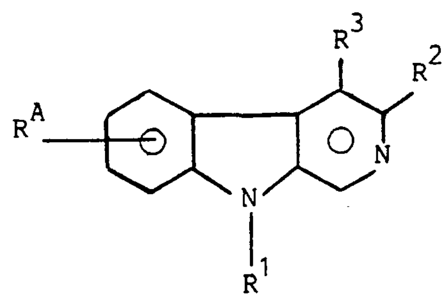

(I)

worin

$R^1$ Wasserstoff oder eine Schutzgruppe,

$R^2$ $-CH=CR^4_2$ oder $-C\equiv CR^4$ mit $R^4$ in der Bedeutung von Wasserstoff oder Halogen,

$R^3$ Wasserstoff, niederes Alkyl oder niederes Alkoxyalkyl und

$R^A$ Wasserstoff, $NR^5R^6$, niederes Alkyl, das gegebenenfalls substituiert ist mit gegebenenfalls substituiertem Aryl, niederem Alkoxy oder

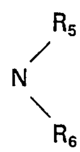

,

wobei $R^5$ und $R^6$ gleich oder verschieden sein können und jeweils Wasserstoff, niederes Alkyl oder gemeinsam mit Stickstoffatom einen 5—6 gliedrigen Ring bedeuten, der ein weiteres Heteroatom enthalten kann, oder

$OR^7$, wobei $R^7$ niederes Alkyl, gegebenenfalls substituiertes Phenyl oder Phenyl-$C_{1-2}$-Alkyl bedeutet und

jede Verbindung 1 oder mehrere nicht Wasserstoff bedeutet $R^A$-Rest enthalten kann.

Die neuen 3-Vinyl- oder 3-Ethinyl-β-carbolinderivate der allgemeinen Formel I können im A-Ring in Position 5—8 ein- oder mehrfach substituiert sein, wobei die Substitution in 5-, 6- oder 7-Stellung bevorzugt ist.

Unter neiderem Alkyl sind sowohl gerad- als auch verzweigtkettige Reste mit $C_1$—$C_4$ Kohlenstoffatomen wie Methyl, Ethyl, n-Propyl, iso-Propyl, n-Butyl, iso-Butyl, tert. Butyl zu verstehen.

Als Halogen sind Brom und Chlor bevorzugt.

Bilden $R^5$ und $R^6$ mit dem Stickstoffatom gemeinsam einen Heterocyclus so kann dieser ein weiteres Stickstoff-, Sauerstoff- oder Schwefelatom enthalten. Beispielsweise sind die folgenden Heterocyclen als Reste geeignet: Pyrrolidin, Piperazin, Morpholin, Thiomorpholin und Piperidin.

Der Phenylrest $R^7$ kann 1- oder mehrfach substituiert sein durch Halogen wie beispielsweise durch Fluor, Chlor, Brom oder Jod, durch $C_{1-2}$Alkyle oder durch $C_{1-2}$ Alkoxygruppen.

Es ist bekannt, daß bestimmte Stellen im zentralen Nervensystem von Vertebraten eine hohe spezifische Affinität für die Bindung von 1.4- und 1.5-Benzodiazepinen aufweisen (Squires, R. F. und Braestrup, C., Nature (London) 266 (1977) 734). Die Bindungsstellen werden Benzodiazepin-Rezeptoren genannt.

Es wurde gefunden, daß die erfindungsgemäßen substituierten β-Carboline, obwohl sie sich in ihrer chemischen Struktur von den Benzodiazepinen stark unterscheiden, überraschenderweise eine starke Affinität und Spezifität für die Bindung an die Benzodiazepin-Rezeptoren zeigen, indem sie radioaktivmarkiertes Flunitrazepam von diesen Benzodiazepin-Rezeptoren verdrängen.

Die Verdrängungsaktivität der erfindungsgemäßen Verbindungen wird als $IC_{50}$- bzw. $ED_{50}$-Wert angegeben. Der $IC_{50}$-Wert gibt die Konzentration an, die eine 50%ige Verdrängung der spezifischen Bindung von $^3$H-Flunitrazepam (1,0 nM, 0°C) in Proben mit einem Gesamtvolumen von 0,55 ml einer Suspension von Hirnmembranen, z.B. von Ratten, bewerkt.

Die Verdrängungsaktivität wird im in-vitro Test wie folgt bestimmt: 0,5 ml einer Suspension von unbehandeltem Ratten-Vorderhirn in 25 mM $KH_2PO_4$, pH = 7,1 (5—10 mg Gewebe/Probe) wird für 40—60 Minuten bei 0°C zusammen mit $^3$H-Diazepam (spezifische Aktivität 14,4 Ci/mMol, 1,9 nM) oder $^3$H-Flunitrazepam (spezifische Aktivität 87 Ci/mMol, 1,0 nM) inkubiert. Nach Inkubation wird die Suspension

durch eine Glasfritte filtriert, der Rückstand 2 mal mit kalter Pufferlösung gewaschen und die Radioaktivität am Scintillationszähler gemessen.

Der Versuch wird dann wiederholt, jedoch so, daß vor der Zugabe des radioaktiv-markierten Benzodiazepins eine bestimmte Menge oder eine überschüssige Menge der Verbindung, deren Verdrängungsaktivität zu bestimmen ist, zugesetzt wird. Auf Grundlage der erhaltenen Werte wird der $IC_{50}$-Wert berechnet.

Der $ED_{50}$-Wert stellt die Dosis einer Versuchssubstanz dar, die eine Reduktion der spezifischen Bindung des Flunitrazepams an dem Benzodiazepin-Rezeptor in einem lebenden Gehirn auf 50% des Kontrollwertes bewirkt.

Der in-vivo Test wird wie folgt ausgeführt:

Gruppen von Mäusen wird die Versuchssubstanz in unterschiedlichen Dosen und normalerweise subcutan injiziert. Nach 15 Minuten wird den Mäusen das $^3$H-Flunitrazepam intravenös verabreicht. Nach weiteren 20 Minuten werden die Mäuse getötet, ihr Vorderhirn entfernt und die an die Hirnmembranen spezifisch gebundene Radioaktivität durch Scintillationszählung gemessen. Der $ED_{50}$-Wert wird mit Hilfe der Dosis/Wirkungs-Kurven bestimmt.

Die erfindungsgemäßen Verbindungen zeigen in pharmakologischen Tests anti-aggressive, anxiolytische und anti-konvulsive Wirksamkeit. Sie eignen sich insbesondere als Anxiolytikum und antiepileptikum. Die erfindungsgemäßen Verbindungen erscheinen aufgrund ihrer biologisch Wirksamkeit als Psychopharmaka für die Humanmedizin geeignet. Sie können hierzu psychopharmazeutischen Präparationen, zum Beispiel für die orale und parenterale Anwendung, formuliert angewendet werden.

Als Formulierungshilfsstoffe sind physiologisch verträgliche organische und anorganische Trägersubstanzen geeignet, die gegenüber den erfindungsgemäßen Verbindungen inert sind.

Als Trägersubstanzen seien beispielsweise genannt Wasser, Salzlösungen, Alkohole, Polyäthylenglykole, polyhydroxyethoxyliertes Rizinusöl, Gelatine, Lactose, Amylose, Magnesiumstearat, Talkum, Kieselsäure, Fettsäuremono- und diglyceride, Pentaerythritolfettsäureester, Hydroxymethylcellulose und Polyvinylpyrrolidon.

Die pharmazeutischen Präparationen können sterilisiert und/oder mit Hilfsstoffen, wie Schmiermittel, Konservierungsstoffen, Stabilisatoren, Netzmittel, Emulgatoren, Puffermittel und Farbstoffen, versetzt werden.

Für die parenterale Anwendung sind insbesondere Injektionslösungen oder Suspensionen, insbesondere wäßrige Lösungen der aktiven Verbindungen in polyhydroxyethoxyliertem Rizinusöl, geeignet.

Für die orale Anwendung sind insbesondere Tabletten, Dragées oder Kapseln mit Talkum und/oder einem Kohlenwasserstoffräger oder — binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt wird.

Die erfindungsgemäßen Verbindungen werden in einer Dosiseinheit von 0,05 bis 100 mg aktiver Substanz in einem physiologisch verträglichen Träger eingebracht.

Die erfindungsgemäßen Verbindungen werden in einer Dosis von 0,1 bis 300 mg/Tag, vorzugsweise 1—30 mg/Tag, angewendet.

Die Herstellung der erfindungsgemäßen Verbindungen der allgemeinen Formel I erfolgt nach an sich bekannten Methoden.

Beispielsweise erfolgt die Herstellung der Verbindungen der allgemeinen Formel I, dadurch, daß man eine Verbindung der allgemeinen Formel II

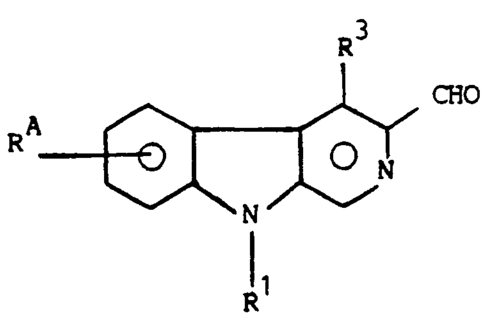

(II)

worin

$R^3$ und $R^A$ die oben genannte Bedeutung haben und

$R^1$ eine Schutzgruppe darstellt, mit einem Wittigreagenz umsetzt zu einer Verbindung der allgemeinen Formel Ia

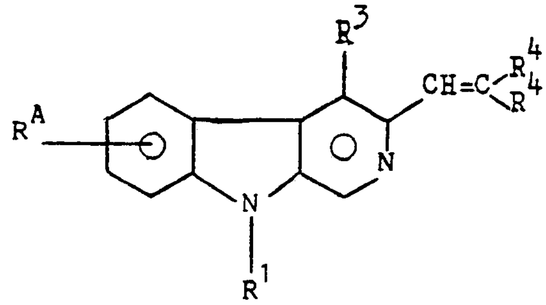

(Ia)

worin

$R^3$, $R^4$ und $R^A$ die oben genannte Bedeutung haben und

$R^1$ eine Schutzgruppe darstellt

und gegebenenfalls anschließend die Schutzgruppe abspaltet oder, falls $R^4$ Halogen bedeutet, mit Basen umsetzt und die so erhaltenen 3-Halogenethinyl-β-carbolin-derivate enthalogeniert und gewünschtenfalls hydriert.

Vor der Durchführung der Wittig-Reaktion ist es vorteilhaft, im Molekül vorhandene Protonendonatoren nach den üblichen Verfahren gegen Schutzgruppen auszutauschen. Als Schutzgruppen sind alle üblicherweise verwendeten Schutzgruppen geeignet wie beispielsweise ein Alkyl-, Acyl-, Aralkyl-, Arylsulfonyl- oder Silylrest, wobei der Arylsulfonyl- oder Trialkylsilylrest, insbesondere der Tosylrest oder der tert.-Butyl-dimethyl-silyl-rest, bevorzugt sind.

Zur Einführung der Vinylgruppe in 3-Stellung werden die üblichen Wittig-Reagenzen verwendet wie beispielsweise Triphenylphosphin/Tetrahalogenmethan oder Alkyl-triphenyl-phosphononiumhalogenide, wobei Halogen bevorzugt Chlor oder Brom beeinhaltet.

Sollen Verbindungen der allgemeinen Formel Ia mit $R^4$ in der Bedeutung von Halogen hergestellt werden, so setzt man den Aldehyd der allgemeinen Formal II mit Triphenylphosphin und Tetrahalogenmethan um.

Die Reaktion wird in inerten polaren Lösungsmitteln bei Temperaturen von −50°C bis zur Siedetemperatur des Reaktionsgemisches bevorzugt bei Temperaturen von −20°C bis +50°C durchgeführt. Als geeignete Lösungsmittel seie beispielsweise genannt: chlorierte Kohlenwasserstoffe wie Dichlormethan, Dichlorethan; Ether wie Diethylether, Tetrahydrofuran, Dioxan; Dimethylformamid; Dimethylsulfoxid.

Die Reaktionszeit beträgt 15—20 Stunden und kann bei Arbeiten unter Ultraschall oder durch Zugabe von Zink deutlich beschleunigt werden auf ca. 6—8 Stunden.

Sollen Verbindungen de allgemeinen Formel Ia mit $R^4$ in der Bedeutung von Wasserstoff hergestellt werden, so setzt man den Aldehyd der allgemeinen Formel II mit Methyl-triphenyl-phosphonium-halogeniden, bevorzugt den Bromiden oder Chloriden, in Gegenwart von Basen um.

Zur Erzeugung des Ylens kann man alle starken, bevorzugt Alkali-organischen, Basen verwenden wie beispielsweise Alkalialkoholate wie Kalium-tert.-butylat, Lithium-organyle wie tert.-Butyllithium oder Lithiumdiisopropylamid, Natrium oder Natriumhydrid/Dimethylsulfoxid u.U, aber auch Kaliumcarbonat oder Natriumhydroxid.

Die oben genannten inerten polaren Lösungsmittel wie auch in besonderen Fällen Kohlenwasserstoffe, zum Beispiel Hexan oder Pentan, können als Lösungsmittel verwendet werden.

Die Umsetzung erfolgt bei Temperaturen von −50°C bis zur Siedetemperatur des Reaktionsgemisches und ist im allgemeinen nach 1—4 Stunden beendet.

Zweckmäßigerweise wird unter Inertgasatmosphäre, beispielsweise unter Stickstoff oder Argon, gearbeitet.

Die Wittig-Reaktion kann in homogener oder heterogener Phase durchgeführt werden. Bei einer Zweiphasenreaktion ist es auch zweckmäßig, einen Phasentransferkatalysator zuzusetzen, beispielsweise einen Kronenäther wie 18-Krone-6, Dicyclohexyl-18-Krone-6, Dibenzo-18-Krone-6 oder Aliquat 336.

Falls vorhanden kann die Schutzgruppe $R^1$ nach den üblichen Methoden beispielsweise durch Behandeln mit Basen wie Natrium- oder Kaliumalkoholat oder Säuren wie verdünnter Mineralsäure oder Trifluoressigsäure in inerten Lösungsmitteln wie Alkoholen, Kohlenwasserstoffen u.a. bei Raumtemperatur abgespalten werden.

Aus der geminalen Dihalogenverbindung der allgemeinen Formel Ia kann durch Umsetzung mit Basen bei erhöhter Temperatur (bevorzugt 20°C bis 100°C) Halogenwasserstoff abgespalten werden. Im allgemeinen ist die Reaktion nach 2—6 Stunden beendet.

Für die Halogenwasserstoffabspaltung können anorganische oder organische Basen verwendet werden wie beispielsweise Kaliumhydroxid, Natriumhydroxid (in fester Form), Alkalialkoholate wie Natrium- oder Kalium-ethylat, -methylat oder-tert.-butylat, alkylierte Amine wie die Hünig Base, cyclische Amine wie 1,5-Diazabicyclo-(5.4.0)-undec-5-en, 1,4-Diazabicyclo-(2.2.2)-octan u.a.

Die Halogenwasserstoffabspaltung kann in allen inerten aprotischen und protische Lösungsmitteln durchgeführt werden. Beispielsweise seien genannt: Ether wie Diethylether, Tetrahydrofuran;

Kohlenwasserstoff wie Hexan, Pentan; Alkohole wie Methanol, Ethanol.

Ist in 9-Stellung eine Schutzgruppe vorhanden, so wird diese unter den Reaktionsbedingung abgespalten.

Auch Zweiphasenreaktionen sind mit Verwendung eines Phasentransferkatalysators wie oben beschrieben möglich.

Die 3-Halogen-ethinyl-β-carbolinderivate der allgemeinen Formel I werden durch Umsetzung mit Lithium-Organylen und anschließender Zersetzung mit Wasser in die 3-Ethinyl-β-carbolinderivate überführt.

Alle bekannten Lithium-Organyle können zur Umsetzung eingesetzt werden, wobei Lithiumphenyl und Lithiumalkyle wie beispielsweise tert.-Butyllithium bevorzugt sind.

Der Halogen-Lithium-Austausch wird bei tiefen Temperaturen (vorzugsweise 0°C bis −90°C) vorgenommen und ist nach 1/2 Stunde bis 2 Stunden beendet. Anschließend wird 1—2 Stunden bei Raumtemperatur nachgerührt.

Zweckmäßigerweise arbeitet man unter Inertgasatmosphäre beispielsweise unter Stickstoff oder Argon.

Für die Enthalogenierung sind aprotische Lösungsmittel wie Ether oder Kohlenwasserstoff geeignet beispielsweise Tetrahydrofuran, Dioxan, Diethylether, Toluol, Hexan u.a.

Die 3-Ethinyl-β-carbolin-derivate können partiell zu den 3-Vinyl-derivaten nach bekannten Verfahren hydriert werden wie beispielsweise in Gegenwart von Lindlar-Katalysatoren oder durch Hydroaluminierung und anschließender protolytischer Spaltung.

Die Hydrierung an Lindlar-Katalysatoren wird, gegebenenfalls unter Zusatz eines Katalysatorgiftes wie beispielsweise Chinolin oder Pyridin, bei Raumtemperatur in einem inerten Lösungsmittel wie beispielsweise Kohlenwasserstoffen oder Alkoholen u.a. vorgenommen.

Die Hydroaluminierung kann man mit Aluminiumorganischen Verbindungen wie zum Beispiel Diisobutylaluminiumhydrid bei Temperaturen von −80°C bis Raumtemperatur und anschließendem Erwärmen auf bis zu 80°C durchführen. Zweckmäßigerweise arbeitet man unter Schutzgasatmosphäre in inerten Lösungsmitteln wie beispielsweise Ethern oder Kohlenwasserstoffen. Die Spaltung wird mit Säuren bevorzugt anorganischen Säuren wie beispielsweise Salzsäure bei Raumtemperatur vorgenommen. Die Ausgangsverbindungen sind bekannt oder können nach bekannten Verfahren hergestellt werden.

Herstellung des Ausgangsmaterials

5-Benzyloxy-4-methoxymethyl-9-tosyl-β-carbolin-3-carbaldehyd

3,9 g 5-Benzyloxy-4-methoxymethyl-β-carbolin-3-carbonsäureethylester werden in 70 ml Dichlormethan mit 1,84 ml Triethylamin und 0,54 g Dimethylaminopyridin gelöst. Die Lösung wird auf 0°C gekühlt und dann portionsweise mit 2,54 g Tosylchlorid versetzt. Nach 2 Stunden Rühren bei Raumtemperatur und Stehen über Nacht wird dreimal mit Natriumhydrogenkarbonatlösung ausgeschüttelt, die organische Phase eingeengt und aus Essigester umkristallisiert. Man erhält 4 g 5-Benzyloxy-4-methoxymethyl-9-tosyl-β-carbolin-3-carbonsäureethylester vom Schmelzpunkt 149—150°C. Diese 4 g werden in 40 ml Tetrahydrofuran unter Argon gelöst und bei Raumtemperatur mit 17 ml einer 1,7 molaren Lösung von Calziumdiisopropoxy-aluminiumhydrid in Tetrahydrofuran (Capal) versetzt; nach einstündigem Erhitzen zum Rückfluß werden nochmals 17 ml Capal zugegeben und 2 Stunden zum Rückfluß erhitzt. Nach Abkühlen wird mit 2n Natronlauge versetzt und mit Essigester ausgeschüttelt. Die organische Phase wird eingeengt und man erhält 3,1 g 5-Benzyloxy-4-methoxymethyl-9-tosyl-3-hydroxymethyl-β-carbolin, die in 57 ml Toluol suspendiert und mit 1 ml Azodicarbonsäureester versetzt werden. Nach zwölfstündigem Erhitzen am Rückfluß wird eingeengt und über Kieselgel mit Cyclohexan: Essigester — 1:1 als Elutionsmittel erhält man 1,8 g 5-Benzyloxy-4-methoxymethyl-9-tosyl-β-carbolin-3-carbaldehyd vom Schmelzpunkt 125—127°C.

In gleicher Weise werden hergestellt:

5-Isopropoxy-4-methyl-9-tosyl-β-carbolin-3-carbaldehyd (172—175°C Fp.).
5-Phenoxy-4-methoxymethyl-9-tosyl-β-carbolin-3-carbaldehyd (150—153°C Fp.).
5-Benzyloxy-9-tosyl-β-carbolin-3-carbaldehyd (185—187° C Fp.).
6-Benzyloxy-4-methoxymethyl-9-tosyl-β-carbolin-3-carbaldehyd (188—190°C Fp.).
6-Phenoxy-4-methoxymethyl-9-tosyl-β-carbolin-3-carbaldehyd.
6-Isopropoxy-4-methoxymethyl-9-tosyl-β-carbolin-3-carbaldehyd.
6-Phenoxy-4-methyl-9-tosyl-β-carbolin-3-carbaldehyd.
6-(4-Chlorphenoxy)-4-methoxymethyl-9-tosyl-β-carbolin-3-carbaldehyd (215—217°C Fp.).
6-Phenoxy-9-tosyl-β-carbolin-3-carbaldehyd.
6,7-Dimethoxy-9-tosyl-4-ethyl-β-carbolin-3-carbaldehyd.
5-(1-Ethoxyethyl)-4-methyl-9-tosyl-β-carbolin-3-carbaldehyd.
5-(Methoxymethyl)-4-methyl-9-tosyl-β-carbolin-3-carbaldehyd.
5-(Morpholinomethyl)-4-methyl-9-tosyl-β-carbolin-3-carbaldehyd.
6-Piperidino-4-methyl-9-tosyl-β-carbolin-3-carbaldehyd.
5-Ethyl-4-methyl-9-tosyl-β-carbolin-3-carbaldehyd.
5(-(4-Fluorbenzyloxy)-4-methoxymethyl-9-tosyl-β-carbolin-3-carbaldehyd (170—172°C Fp.).
5-(4-Chlorphenoxy)-4-methoxymethyl-9-tosyl-β-carbolin-3-carbaldehyd.

Die folgenden Beispiele sollen das erfindungsgemäße Verfahren erläutern.

Beispiel 1

5-Benzyloxy-3-(1,1-dibromvinyl)-4-methoxymethyl-9-tosyl-β-carbolin

2,5 g 5-Benzyloxy-4-methoxymethyl-9-tosyl-β-carbolin-3-carboxaldehyd und 2,6 g Triphenylphosphin werden in 27 ml Dichlormethan gelöst und auf +5°C gekühlt. Zu dieser Lösung tropft man unter Rühren 1,82 g Tetrabrommethan, gelöst in 7 ml Dichlormethan, so, daß die Reaktionstemperatur +5°C nicht überschreitet. Nach beendeter Zugabe wird das Reaktionsgemisch 16 Stunden bei Raumtemperatur gerührt, das Lösungsmittel eingedampft und der Rückstand über Kieselgel mit Dichlormethan/Ethanol = 1000 + 25 als Elutionsmittel chromatographiert. Man erhält 2 g 5-Benzyloxy-3-(1,1-dibromvinyl)-4-methoxymethyl-9-tosyl-β-carbolin vom Schemlzpunkt 149—152°C.

Beispiel 2

Analog Beispiel 1 wurden hergestellt:

5-Isopropoxy-3-(1,1-dibromvinyl)-4-methoxymethyl-9-tosyl-β-carbolin.
5. Phenoxy-3-(1,1-dibromvinyl)-4-methoxymethyl-9-tosyl-β-carbolin Fp. 179—183°C.
5-(4-Chlorphenoxy)-3-(1,1-dibromvinyl)-4-methoxymethyl-9-tosyl-β-carbolin Fp. 165—167°C.
5-Benzyloxy-3-(1,1-dibromvinyl)-4-methyl-9-tosyl-β-carbolin.
5-Isopropoxy-3-(1,1-dibromvinyl)-4-methyl-9-tosyl-β-carbolin Fp. 168—171°C.
5-Phenoxy-3-(1,1-dibromvinyl)-4-methyl-9-tosyl-β-carbolin.
5-Benzyloxy-3-(1,1-dibromvinyl)-9-tosyl-β-carbolin Fp. 160—162°C.
6-Benzyloxy-3-(1,1-dibromvinyl)-4-methoxymethyl-9-tosyl-β-carbolin.
6-Phenoxy-3-(1,1-dibromvinyl)-4-methoxymethyl-9-tosyl-β-carbolin.
6-Isopropoxy-3-(1,1-dibromvinyl)-4-methoxymethyl-9-tosyl-β-carbolin.
6-Phenoxy-3-(1,1-dibromvinyl)-4-methyl-9-tosyl-β-carbolin.
6-(4-Chlorphenoxy)-3-(1,1-dibromvinyl)-4-methyl-9-tosyl-β-carbolin.
5-(4-Fluorbenzyloxy)-3-(1,1-dibromvinyl)-4-methoxymethyl-9-tosyl-β-carbolin (144—145°C Fp.).
6-Phenoxy-3-(1,1-dibromvinyl)-9-tosyl-β-carbolin.
6,7-Dimethoxy-3-(1,1-dibromvinyl)-4-ethyl-9-tosyl-β-carbolin.
5-(1-Ethoxyethyl)-4-methyl-3-(1,1-dibromvinyl)-9-tosyl-β-carbolin.
5-Methoxymethyl-4-methyl-3-(1,1-dibromvinyl)-9-tosyl-β-carbolin.
5-Morpholinomethyl-4-methyl-3-(1,1-dibromvinyl)-9-tosyl-β-carbolin.
6-Piperidino-4-methyl-3-(1,1-dibromvinyl)-9-tosyl-β-carbolin.
5-Ethyl-4-methyl-3-(1,1-dibromvinyl)-9-tosyl-β-carbolin.

Beispiel 3

1-Brom-2-(5-benzyloxy-4-methoxymethyl-β-carbolin-3-yl)acetylen

Eine Lösung von 0,141 g Natrium in 12 ml Methanol wird unter Rühren zu einer LÖsung von 2 g 5-Benzyloxy-3-(1,1-dibromvinyl)-4-methoxymethyl-9-tosyl-β-carbolin in 25 ml Methanol getropft. Die Reaktion wird bei Raumtemperatur 3 Stunden gerührt und anschließend zur Trockne eingedampft. Der Rückstand wird über Kieselgel mit Dichlormethan/Ethanol = 10/1 als Elutionsmittel chromatographiert. Man erhält 0,7 g 1-Brom-2-(5-benzyloxy-4-methoxymethyl-β-carbolin-3-yl)-acetylen von Schlemzpunkt 160°C (Zersetzung).

Beispiel 4

Analog Beispiel 3 wurden hergestellt:

1-Brom-2-(5-isopropoxy-4-methoxymethyl-β-carbolin-3-yl)-acetylen.
1-Brom-2-(5-phenoxy-4-methoxymethyl-β-carbolin-3-yl)acetylen 210—212°C Fp. (Zersetzung).
1-Brom-2-[5-(4-Chlorphenoxy)-4-methoxymethyl-β-carbolin-3-yl]-acetylen 207—209°C Fp. (Zersetzung).
1-Brom-2-(5-benzyloxy-4-methyl-β-carbolin-3-yl-)-acetylen.
1-Brom-2-(5-isopropoxy-4-methyl-β-carbolin-3-yl)-acetylen 170°C Fp. (Zersetzung).
1-Brom-2-[5-(4-Fluorbenzyloxy)-4-methoxymethyl-β-carbolin-3-yl)]-acetetylen 151—153°C Fp. (Zersetzung).
1-Brom-2-(5-phenoxy-4-methyl-β-carbolin-3-yl)-acetylen 188°C Fp. (Zersetzung).
1-Brom-2-(5-benzyloxy-β-carbolin-3-yl)-acetylen.
1-Brom-2-(6-benzyloxy-4-methoxymethyl-β-carbolin-3-yl)acetylen 177—180°C Fp. (Zersetzung).
1-Brom-2-(6-phenoxy-4-methoxymethyl-β-carbolin-3-yl)-acetylen.
1-Brom-2-(6-isopropoxy-4-methoxymethyl-β-carbolin-3-yl)-acetylen.
1-Brom-2-(6-phenoxy-4-methyl-β-carbolin-3-yl)-acetylen.
1-Brom-2-[5-(4-Chlorphenoxy)-4-methyl-β-carbolin-3-yl]-acetylen.
1-Brom-2-(6-phenoxy-β-carbolin-3-yl)-acetylen.
1-Brom-2-(6,7-dimethoxy-4-ethyl-β-carbolin-3-yl)-acetylen.
1-Brom-2-[5-(1-Ethoxyethyl)-4-methyl-β-carbolin-3-yl]-acetylen.
1-Brom-2-(5-methoxymethyl-4-methyl-β-carbolin-3-yl)-acetylen.
1-Brom-2-(5-morpholinomethyl-4-methyl-β-carbolin-3-yl)-acetylen.
1-Brom-2-(6-piperidino-4-methyl-β-carbolin-3-yl)acetylen.
1-Brom-2-(5-Ethyl-4-methyl-β-carbolin-3-yl)-acetylen.

Beispiel 5

5-Benzyloxy-3-ethinyl-4-methoxymethyl-β-carbolin

Eine Lösung von 0,164 g 1-Brom-2-(5-benzyloxy-4-methoxymethyl-β-carbolin-3-yl-acetylen in 5 ml abs. Tetrahydrofuran wird unter $N_2$-Atmosphäre auf −78°C gekühlt und mit 0,46 ml einer 1,4 molaren Lösung tert.-Butyllithium in Pentan versetzt. Nach 1 Stunde rühren bei −78°C wird die Reaktionsmischung auf Raumtemperatur erwärmt und weitere 1,5-Stunden gerührt. Danach wird vorsichtig mit Wasser zersetzt und mit Dichlormethan extrahiert. Die organischen Basen werden vereinigt, getrocknet und das Lösungsmittel eingedampft. Der Rückstand wird über Kieselgel mit Aceton/Hexan = 1 + 1 als Elutionsmittel chromatographiert. Man erhält 0,056 g 5-Benzyloxy-3-ethinyl-4-methoxymethyl-β-carbolin von Schmelzpunkt 207—209°C.

Beispiel 6

Analog Beispiel 5 wurden hergestellt:

5-Isopropoxy-3-ethinyl-4-methoxymethyl-β-carbolin.
5-Phenoxy-3-ethinyl-4-methoxymethyl-β-carbolin 115°C Fp. (Zersetzung).
5-(4-Chlorphenoxy)-3-ethinyl-4-methoxymethyl-β-carbolin 260—263°C Fp. (Zersetzung).
5-Benzyloxy-3-ethinyl-4-methyl-β-carbolin.
5-Isopropoxy -3-ethinyl-4-methyl-β-carbolin Fp. 205—207°C.
5-Phenoxy-3-ethinyl-4-methyl-β-carbolin.
5-Benzyloxy-3-ethinyl-β-carbolin Fp. 206—210°C.
6-Benzyloxy-3-ethinyl-4-methoxymethyl-β-carbolin Fp. 205—209°C.
6-Phenoxy-3-ethinyl-4-methoxymethyl-β-carbolin.
6-Isopropoxy-3-ethinyl-4-methoxymethyl-β-carbolin.
6-Phenoxy-3-ethinyl-4-methyl-β-carbolin.
6-(4-Chlorphenoxy)-3-ethinyl-4-methyl-β-carbolin.
6-Phenoxy-3-ethinyl-β-carbolin.
6,7-Dimethoxy-3-ethinyl-4-ethyl-β-carbolin.
5-(1-Ethoxyethyl)-4-methyl-3-ethinyl-β-carbolin.
5-Methoxymethyl-4-methyl-3-ethinyl-β-carbolin.
5-Morpholinomethyl-4-methyl-3-ethinyl-β-carbolin.
6-Piperidino-4-methyl-3-ethinyl-β-carbolin.
5-Ethyl-4-methyl-3-ethinyl-β-carbolin.
5-(4-Fluorbenzyloxy)-3-ethinyl-4-methoxymethyl-β-carbolin 181—184°C Fp. (Zersetzung).

Beispiel 7

5-Benzyloxy-4-methoxymethyl-9-tosyl-3-vinyl-β-carbolin

1,0 g Methyltriphenylphosphoniumbromid + Natriumamid (Zustand-Ylid.) werden in 8 ml absolutem Tetrahydrofuran bei RT unter Argon 15 Min. gerührt.

Dazu gibt man 500 mg 5-Benzyloxy-4-methoxymethyl-9-tosyl-β-carbolin-3-carbaldehyd und erhitzt das Reaktionsgemisch 4 Stunden am Rückfluß. Nach Eindampfen des Lösungsmittels wird der Rückstand über Kieselgel mit Cyclohexan/Essigester = 8 + 2 als Elutionsmittel chromatographiert. Man erhält 0,153 mg 5-Benzyloxy-4-methoxymethyl-9-tosyl-3-vinyl-β-carbolin von Schmelzpunkt 141—143°C.

Beispiel 8

5-Benzyloxy-4-methoxymethyl-3-vinyl-β-carbolin

Eine Lösung von 0,23 g Natrium in 10 ml Methanol wird unter Rühren zu einer Suspension von 0.5 g 5-Benzyloxy-4-methoxymethyl-9-tosyl-3-vinyl-β-carbolin in 20 ml Methanol getropft. Nash 72 Stunden Rühren bei RT wird zur Trockene eingedampft und der Rückstand über Kieselgel mit Dichlormethan/Ethanol = 10/1 als Elutionsmittel chromatografiert. Man erhält 0,135 g 5-Benzyloxy-4-methoxymethyl-3-vinyl-β-carbolin vom Schmelzpunkt 183—185°C.

**Patentansprüche**

1. Verbindungen der allgemeinen Formel I

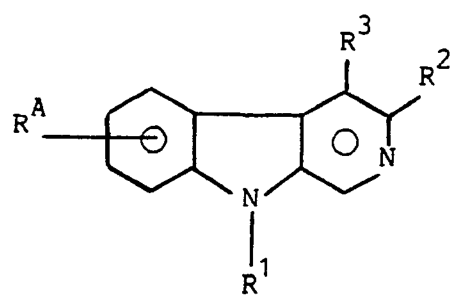

(I)

worin

$R^1$ Wasserstoff oder eine Schutzgruppe,

$R^2$ —CH=$CR^4_2$ oder —C≡$CR^4$ mit $R^4$ in der Bedeutung von Wasserstoff oder Halogen,

$R^3$ Wasserstoff, $C_{1-4}$-Alkyl oder $C_{1-4}$-Alkoxyalkyl und

$R^A$ Wasserstoff, $NR^5R^6$, $OR^7$ oder $C_{1-4}$-Alkyl, das gegebenenfalls substituiert ist mit gegebenenfalls substituiertem Phenyl, $C_{1-4}$-Alkoxy oder $NR^5R^6$, bedeutet, wobei

$R^5$ und $R^6$ gleich oder verschieden sein können und jeweils Wasserstoff, $C_{1-4}$-Alkyl oder gemeinsam mit dem Stickstoffatom Pyrrolidin, Piperazin, Morpholin, Thiomorpholin oder Piperidin bedeuten und

$R^7$ $C_{1-4}$-Alkyl, gegebenenfalls mit Halogen, $C_{1-2}$-Alkyl oder $C_{1-2}$-Alkoxy substituiertes Phenyl oder Phenyl-$C_{1-2}$-Alkyl bedeutet und jede Verbindung 1 oder mehrere nicht Wasserstoff bedeutende $R^A$-Reste enthalten kann.

2. 1-Brom-2-(5-isopropoxy-4-methoxymethyl-β-carbolin-3-yl)-acetylen
1-Brom-2-(5-phenoxy-4-methoxymethyl-β-carbolin-3-yl)-acetylen
1-Brom-2-[5-(4-Chlorphenoxy)-4-methoxymethyl-β-carbolin-3-yl]-acetylen
1-Brom-2-(5-isopropoxy-4-methyl-β-carbolin-3-yl)-acetylen
1-Brom-2-(6-benzyloxy-4-methoxymethyl-β-carbolin-3-yl)-acetylen
1-Brom-2-(6-phenoxy-4-methoxymethyl-β-carbolin-3-yl)-acetylen
1-Brom-2-(6-isopropoxy-4-methoxymethyl-β-carbolin-3-yl)-acetylen
1-Brom-2-(5-benzyloxy-4-methoxymethyl-β-carbolin-3-yl)-acetylen

3. 1-Brom-2-[5-(1-ethoxyethyl)-4-methyl-β-carbolin-3-yl)-acetylen
1-Brom-2-(5-methoxymethyl-4-methyl-β-carbolin-3-yl)-acetylen
1-Brom-2-(5-morpholinomethyl-4-methyl-β-carbolin-3-yl)-acetylen

4. 1-Brom-2-(6-piperidino-4-methyl-β-carbolin-3-yl)-acetylen

5. 5-Isopropoxy-3-ethinyl-4-methoxymethyl-β-carbolin
5-Phenoxy-3-ethinyl-4-methoxymethyl-β-carbolin
5-(4-Chlorphenoxy)-3-ethinyl-4-methoxymethyl-β-carbolin
5-Isopropoxy-3-ethinyl-4-methyl-β-carbolin
6-Benzyloxy-3-ethinyl-4-methoxymethyl-β-carbolin
6-Phenoxy-3-ethinyl-4-methoxymethyl-β-carbolin
6-Iisopropoxy-3-ethinyl-4-methoxymethyl-β-carbolin
6-(4-Chlorphenoxy)-3-ethinyl-4-methyl-β-carbolin
5-Benzyloxy-3-ethinyl-4-methoxymethyl-β-carbolin

6. 5-Methoxymethyl-4-methyl-3-ethinyl-β-carbolin
5-Morpholinomethyl-4-methoxy-3-ethinyl-β-carbolin

7. 3-Vinyl-5-benzyloxy-4-methoxymethyl-β-carbolin

8. Verfahren zur Herstellung der Verbindungen der allgemeinen Formel I, dadurch gekennzeichnet, daß man eine Verbindung der allgemeinen Formel II

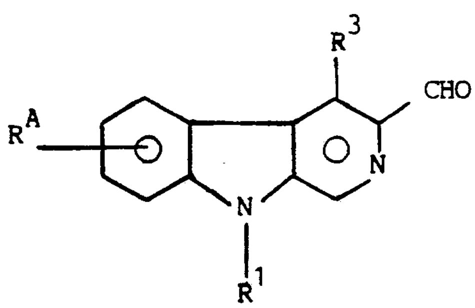

(II)

worin

$R^3$ und $R^A$ die oben genannte Bedeutung haben und

$R^1$ eine Schutzgruppe darstellt, mit einem Wittigreagenz umsetzt zu einer Verbindung der allgemeinen Formel Ia

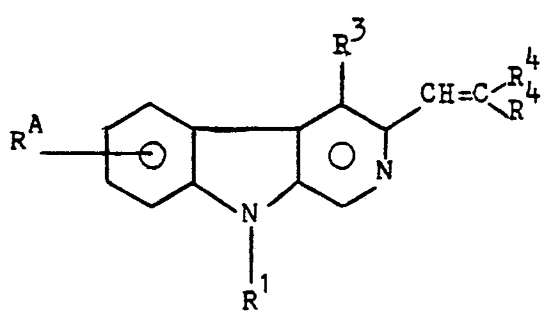

(Ia)

worin

$R^3$, $R^4$ und $R^A$ die oben genannte Bedeutung haben und

$R^1$ eine Schutzgruppe darstellt

und gegebenenfalls anschließend die Schutzgruppe abspaltet oder, falls $R^4$ Halogen bedeutet, mit Basen umsetzt und die so erhaltenen 3-Halogenethinyl-β-carbolin-derivate enthalogeniert und gewünschtenfalls hydriert.

9. Verwendung der Verbindungen gemäß den Ansprüchen 1 bis 7 als Arzneimittel.

**Revendications**

1. Composés répondant à la formule générale I:

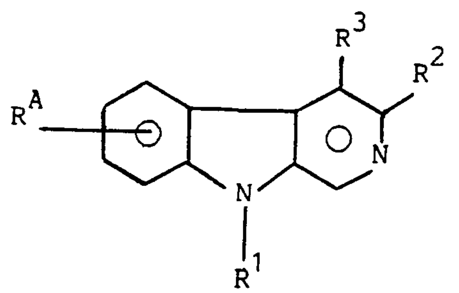

(I)

dans laquelle:

$R^1$ représente l'hydrogène ou un radical protecteur,

$R^2$ représente un radical $-CH=CH_2^4$ ou un rdical $-C{\equiv}CR^4$ dans lesquels $R^4$ désigne l'hydrogène ou un halogène,

$R^3$ représente l'hydrogène, un alkyle en $C_1-C_4$ ou un alcoxyalkyle en $C_1-C_4$ et

$R^A$ représente l'hydrogène, un radical $NR^5R^6$, un radical $OR^7$ ou un radical alkyle en $C_1-C_4$ éventuellement porteur d'un phényle, lui-même éventuellement substitué, d'un alcoxy en $C_1-C_4$ ou d'un radical $NR^5R^6$,

$R^5$ et $R^6$ représentant chacun, indépendamment l'un de l'autre, l'hydrogène ou un alkyle en $C_1-C_4$ ou formant ensemble, et avec l'atome d'azote, un cyle de pyrrolidine, de pipérazine, de morpholine, de thiomorpholine ou de pipéridine, et

$R^7$ représentant un alkyle en $C_1-C_4$, un phényle éventuellement porteur d'un halogène, d'un alkyle en $C_1$ ou $C_2$ ou d'un alcoxy en $C_1$ ou $C_2$, ou un phénylalkyle à alkyle en $C_1$ ou $C_2$,

et dans laquelle il peut y avoir un ou plusieurs radicaux $R^A$ autres que l'hydrogène.

2. Bromo-1 (isopropoxy-5 méthoxyméthyl-4 β-carbolinyl-3)-2 acétylène,
bromo-1 (phénoxy-5 méthoxyméthyl-4 β-carbolinyl-3)-2 acétylène,
bromo-1 [(chloro-4 phénoxy)-5 méthoxyméthyl-4 β-carbolinyl-3]-2 acétylène,
bromo-1 (isopropoxy-5 méthyl-4 β-carbolinyl-3)-2 acétylène,
bromo-1 (benzyloxy-6 méthoxyméthyl-4 β-carbolinyl-3)-2 acétylène,
bromo-1 (phénoxy-6 méthoxyméthyl-4 β-carbolinyl-3)-2 acétylène,
bromo-1 (isopropoxy-6 méthoxyméthyl-4 β-carbolinyl-3)-2 acétylène,
bromo-1 (benzyloxy-5 méthoxyméthyl-4 β-carbolinyl-3)-2 acétylène,

3. Bromo-1 [(éthoxy-1 éthyl)-5 méthyl-4 β-carbolinyl-3]-2 acétylène,
bromo-1 (méthoxyméthyl-5 méthyl-4 β-carbolinyl-3)-2 acétylène,
bromo-1 (morpholinométhyl-5 méthyl-4 β-carbolinyl-3)-2 acétylène,

4. Bromo-1 (pipéridino-6 méthyl-4 β-carbolinyl-3)-2 acétylène.

5. Isopropoxy-5 éthynyl-3 méthoxyméthyl-4 β-carboline,
phénoxy-5 éthynyl-3 méthoxyméthyl-4 β-carboline,
(chloro-4 phénoxy)-5 éthynyl-3 méthoxyméthyl-4 β-carboline,
benzyloxy-6 éthynyl-3 méthoxyméthyl-4 β-carboline,
phénoxy-6 éthynyl-3 méthoxyméthyl-4 β-carboline,
isopropoxy-6 éthynyl-3 méthoxyméthyl-4 β-carboline,
(chloro-4 phénoxy)-6 éthynyl-3 méthyl-4 β-carboline,
phénoxy-6 éthynyl-3 méthyl-4 β-carboline,
benzyloxy-5 éthynyl-3 méthoxyméthyl-4 β-carboline.

6. Méthoxyméthyl-5 méthyl-4 éthynyl-3 β-carboline,
morpholinomethyl-5 méthyl-4 éthynyl-3 β-carboline.

7. Vinyl-3 benzyloxy-5 méthoxyméthyl-4 β-carboline.

8. Procédé pour préparer les composés de formule générale I, procédé caractérisé en ce qu'on fait réagir un composé répondant à la formule générale II:

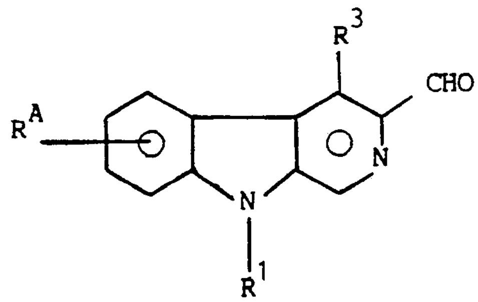

(II)

dans laquelle:

$R^3$ et $R^A$ ont les significations qui leur ont été données ci-dessus et

$R^1$ représente un radical protecteur,

avec un réactif de Wittig de manière à obtenir un composé répondant à la formule générale Ia:

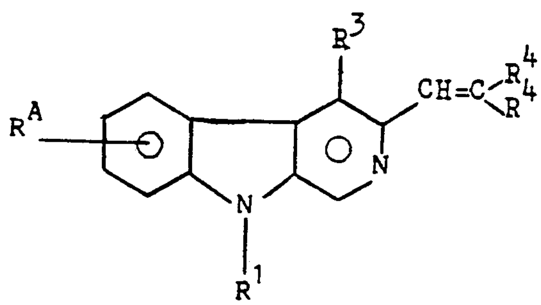

(Ia)

dans laquelle

$R^3$, $R^4$ et $R^A$ ont les significations qui leur ont été données ci-dessus et

$R^1$ représente un radical protecteur,

puis, le cas échéant, on élimine le radical protecteur ou, lorsque $R^4$ représente un halogène, on fait réagir avec des basis et on déshalogène les halogénéthynyl-3 β-carbolines ainsi obtenues et on hydrogène éventuellement.

9. Application des composés selon les revendications 1 à 7 comme médicaments.

**Claims**

1. Compounds of the general formula I

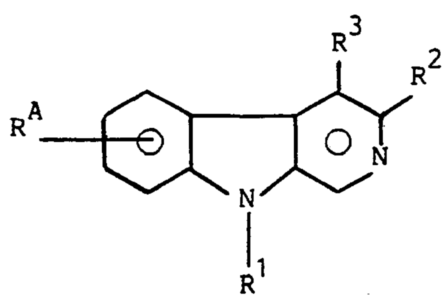

(I)

in which

$R^1$ represents hydrogen or a protecting group,

$R^2$ represents $-CH=CR^4_2$ or $-C\equiv CR^4$ wherein $R^4$ represents hydrogen or halogen,

$R^3$ represents hydrogen, $C_{1-4}$-alkyl or $C_{1-4}$-alkoxyalkyl and

$R^A$ represents hydrogen, $NR^5R^6$, $OR^7$ or $C_{1-4}$-alkyl that is optionally substituted by optionally substituted phenyl, $C_{1-4}$-alkoxy or by $NR^5R^6$ wherein

$R^5$ and $R^6$ may be the same or different and each represents hydrogen or $C_{1-4}$-alkyl, or $R^5$ and $R^6$ together with the nitrogen atom represent pyrrolidine, piperazine, morpholine, thiomorpholine or piperidine, and

$R^7$ represents $C_{1-4}$-alkyl, or phenyl or phenyl-$C_{1-2}$-alkyl optionally substituted by halogen, $C_{1-2}$-alkyl or by $C_{1-2}$-alkoxy,

and each compound may contain one or more $R^A$ radicals with a meaning other than hydrogen.

2. 1-Bromo-2-(5-isopropoxy-4-methoxymethyl-β-carbolin-3-yl)-acetylene
1-bromo-2-(5-phenoxy-4-methoxymethyl-β-carbolin-3-yl)-acetylene
1-bromo-2-[5-(4-chlorophenoxy)-4-methoxymethyl-β-carbolin-3-yl]-acetylene
1-bromo-2-(5-isopropoxy-4-methyl-β-carbolin-3-yl)-acetylene
1-bromo-2-(6-benzyloxy-4-methoxymethyl-β-carbolin-3-yl)-acetylene
1-bromo-2-(6-phenoxy-4-methoxymethyl-β-carbolin-3-yl)-acetylene
1-bromo-2-(6-isopropoxy-4-methoxymethyl-β-carbolin-3-yl)-acetylene
1-bromo-2-(5-benzyloxy-4-methoxymethyl-β-carbolin-3-yl)-acetylene

3. 1-Bromo-2-[5-(1-ethoxyethyl)-4-methyl-β-carbolin-3-yl)-acetylene
1-bromo-2-(5-methoxymethyl-4-methyl-β-carbolin-3-yl)-acetylene
1-bromo-2-(5-morpholinomethyl-4-methyl-β-carbolin-3-yl)-acetylene

4. 1-Bromo-2-(6-piperidino-4-methyl-β-carbolin-3-yl)-acetylene

5. 5-Isopropoxy-3-ethynyl-4-methoxymethyl-β-carboline
5-phenoxy-3-ethynyl-4-methoxymethyl-β-carboline
5-(4-chlorophenoxy)-3-ethynyl-4-methoxymethyl-β-carboline
5-isopropoxy-3-ethynyl-4-methyl-β-carboline
6-benzyloxy-3-ethynyl-4-methoxymethyl-β-carboline
6-phenoxy-3-ethynyl-4-methoxymethyl-β-carboline
6-isopropoxy-3-ethynyl-4-methoxymethyl-β-carboline
6-4-chlorophenoxy)-3-ethynyl-4-methyl-β-carboline
5-benzyloxy-3-ethynyl-4-methoxymethyl-β-carboline

6. 5-Methoxymethyl-4-methyl-3-ethynyl-β-carboline
5-morpholinomethyl-4-methyl-3-ethynyl-β-carboline

7. 3-Vinyl-5-benzyloxy-4-methoxymethyl-β-carboline.

8. A process for the preparation of the compounds of the general formula I, characterised in that a compound of the general formula II

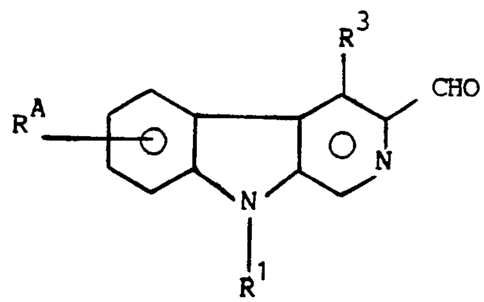

(II)

in which
$R^3$ and $R^A$ are as defined hereinbefore and
$R^1$ represents a protecting group, is reacted with a Wittig reagent to form a compound of the general formula Ia

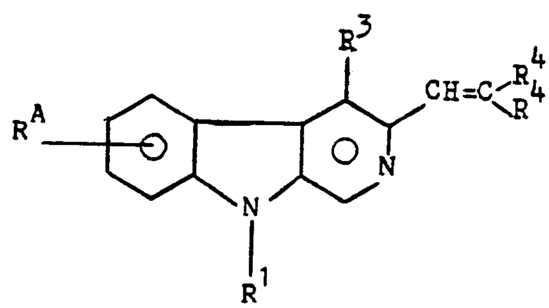

(Ia)

in which
$R^3$, $R^4$ and $R^A$ are as defined hereinbefore and
$R^1$ represents a protecting group,
and optionally then the protecting group is removed or, if $R^4$ represents halogen, the compound of formula (Ia) is reacted with bases, and the 3-haloethynyl-β-carboline derivatives so obtained are dehalogenated and, if desired, hydrogenated.

9. The use of compounds according to claims 1 to 7 as medicaments.